# FASCICULE DE BREVET EUROPEEN

(11) **EP 2 649 936 B1**
(45) Date de publication et mention de la délivrance du brevet: **13.03.2019**
(21) Numéro de dépôt: 13162389.4
(22) Date de dépôt: 04.04.2013
(51) Int. Cl.: A61B 5/0484, H04L 7/00

(54) **Procédé de synchronisation de données et système de transmission de données mettant en oeuvre un tel procédé**
Datensynchronisationsverfahren und Datenübertragungssystem zur Durchführung des Verfahrens
Data synchronization method and data transmission system implementing the method

(30) Priorité: 10.04.2012 FR 1253266
(43) Date de publication de la demande: 16.10.2013
(73) Titulaire: COMMISSARIAT À L'ÉNERGIE ATOMIQUE ET AUX ÉNERGIES ALTERNATIVES, 75015 Paris (FR)
(72) Inventeur: Foerster, Michael, 33170 Gradignan (FR); Charvet, Guillaume, 38360 Sassenage (FR); Porcherot, Jean, 38000 Grenoble (FR)
(74) Mandataire: Gevers & Orès

(56) Documents cités:
- WO-A1-01/06922
- US-B1- 6 366 805

## Description

L'invention porte sur un procédé de synchronisation de données transmises entre au moins un terminal d'émission et un terminal de réception via un canal de transmission à latence non maîtrisée. Elle porte également sur un système de transmission de données permettant la mise en oeuvre d'un tel procédé.

L'invention permet notamment la synchronisation des signaux issus d'un capteur ou d'un ensemble de capteurs reliés à une unité centrale par l'intermédiaire de liaisons sans fil. Elle s'applique notamment à l'acquisition de signaux biologiques, et plus particulièrement neuronaux.

Les systèmes d'acquisition de signaux biologiques existent depuis longtemps, mais ce n'est que depuis peu que la miniaturisation a permis de développer des systèmes d'acquisition sans fil. Les systèmes filaires classiques, comme les bonnets de mesure EEG par exemple, lient physiquement le ou les capteurs à proximité ou en contact avec une personne ou un animal (« le patient ») à une électronique de mesure, qui est à son tour reliée par câble à une unité de traitement des données, qui peut être notamment un ordinateur. Les systèmes sans fil, par contre, intègrent l'électronique d'acquisition sur le lieu de la mesure et transmettent les données à l'unité de traitement de données par un moyen de communication sans fil.

Lors de l'acquisition de signaux biologiques, l'un des principaux sujets d'étude est l'influence de paramètres externes sur les grandeurs mesurées. Par exemple, en neurosciences on cherche à détecter des variations dans les signaux d'électroencéphalographie (EEG) en réponse à un stimulus sensoriel (flash lumineux, appel du prénom, ...). Pour cela il est nécessaire de synchroniser l'instant du stimulus sensoriel et les différents signaux d'EEG, c'est-à-dire les situer sur une base de temps commune.

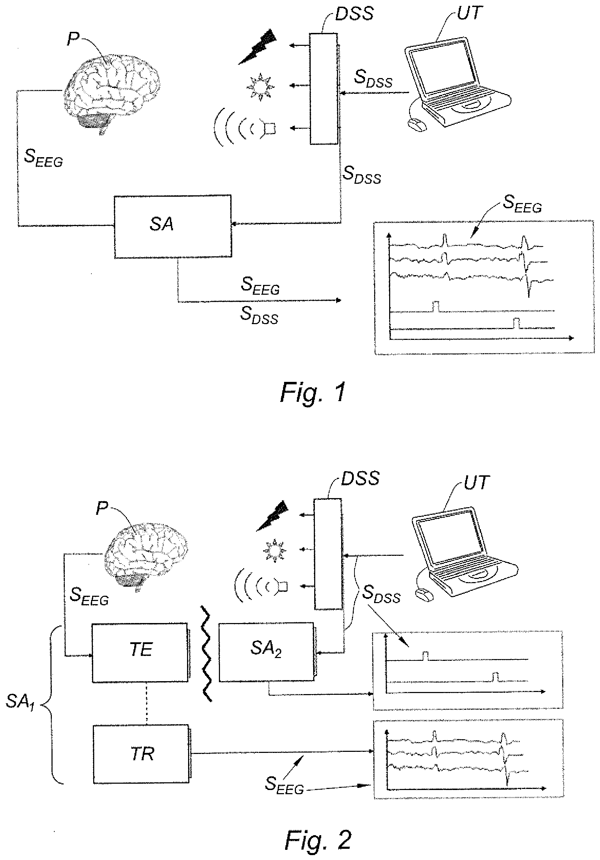

La synchronisation des données ne pose pas de difficulté particulière dans le cas des systèmes conventionnels, dans lesquels les capteurs d'acquisition des signaux sont reliés à l'unité de traitement des données de manière filaire. Il suffit en effet d'utiliser une voie du système d'acquisition comme entrée pour le stimulus. Par exemple, le signal activant un flash peut être redirigé sur l'une des voies d'enregistrement du système d'acquisition. Un système d'acquisition de ce type est illustré sur la figure 1, où la référence P indique un patient subissant un examen d'EEG (seul son cerveau est représenté), DSS un dispositif de génération de stimuli sensoriels (par exemple un flash, un écran, un haut-parleur...), SA un système d'acquisition de données, UT un ordinateur servant à la fois au pilotage du dispositif DSS et au traitement des signaux d'EEG acquis par le système SA. L'ordinateur UT émet un signal de pilotage, S_{DSS}, qui déclenche la génération d'un stimulus sensoriel par le dispositif DSS ; la réponse du cerveau du patient P à ce stimulus est mesurée sous la forme de signaux d'EEG, S_{EEG}. Les signaux S_{DSS} et S_{EEG} sont acquis par des vois de mesure respectives du système SA et transmis à l'ordinateur UT. Les délais de propagation des signaux étant connus, ou en tout cas constants, ce dernier peut aisément les situer sur une base de temps commune.

Le document WO 01/06922 décrit un système de ce type. Dans ce système, un dispositif de stimulation (un magnétoscope) et un convertisseur analogique-numérique de signaux d'EEG sont déclenchés simultanément au moyen d'interrupteurs électroniques respectifs ; puis un ordinateur acquiert tant les signaux vidéos en sortie du magnétoscope que les signaux d'EEG numérisés.

Le document US 6 366 805 B1 décrit un procédé et un système de synchronisation de données de surveillance médicale transmises entre un terminal d'émission et un terminal de réception via un bus filaire formant un canal de transmission à latence suffisamment grande pour être corrigée mais fixe. Le procédé comprend une étape consistant au niveau du terminal d'émission à générer une base de temps commune entre le terminal d'émission et le terminal de réception.

Cette approche ne peut pas être transposée directement aux systèmes d'acquisition sans fil, ou alors seulement au prix d'une synchronisation très imprécise. En effet, les systèmes de transmission de données sans fil présentent le plus souvent un délai (« latence ») qui est relativement important (de l'ordre des centaines de millisecondes), inconnu et - surtout - variable. En effet, pour des raisons de robustesse les protocoles de transmission sans fil mettent généralement en oeuvre des algorithmes sophistiqués de détection et correction des erreurs pouvant impliquer des échanges répétés entre le récepteur et l'émetteur. Le temps nécessaire à transmettre un paquet de données dépend donc à la fois de la qualité de la liaison (rapport signal sur bruit, interférences,...), du débit de transmission et de la charge de travail de l'ordinateur connecté au récepteur. Ainsi, dans les systèmes de communications sans fil, la latence n'est pas maîtrisée (elle est inconnue et variable de manière imprévisible). Cette situation est illustrée par la figure 2, montrant un système d'acquisition comportant deux systèmes d'acquisition séparés : un premier, SA₁, destiné à l'acquisition du signal S_{EEG} et comprenant un terminal d'émission TE relié à un terminal de réception TR par l'intermédiaire d'une liaison sans fil C_{D} ; et un deuxième, SA₂, destiné à l'acquisition du signal S_{DSS}. Les deux systèmes d'acquisition ne communiquent pas entre eux (ou alors par l'intermédiaire d'une liaison sans fil, à latence non maîtrisée). Par conséquent, il n'est pas possible de localiser les signaux S_{EEG} et S_{DSS} sur une base des temps commune, sauf à ignorer la latence de la liaison sans fil (ou à la supposer constante), ce qui implique des erreurs de synchronisation importants.

L'invention vise à surmonter les inconvénients précités de l'art antérieur et à permettre la synchronisation de données transmises entre un terminal d'émission (voire plusieurs) et un terminal de réception via un canal de transmission à latence non maîtrisée.

Un objet de l'invention permettant d'atteindre ce but est un procédé de synchronisation de données transmises entre au moins un terminal d'émission et un terminal de réception via un canal de transmission à latence non maîtrisée et variable définie selon la revendication 1.

Tant ledit canal de transmission que ledit canal de transmission auxiliaire peuvent être des canaux de transmission sans fil. Le canal de transmission auxiliaire peut cependant présenter un faible débit et tolérer un taux d'erreur relativement important, ce qui permet d'avoir recours à un protocole de communication simple, assurant une maîtrise de la latence au détriment de la capacité et de la robustesse.

Selon différents modes de réalisation du procédé de l'invention :
- Ledit ou chaque terminal d'émission peut émettre une pluralité de paquets de données à synchroniser ainsi qu'un dit signal de synchronisation en correspondance de chaque dit paquet de données à synchroniser. Dans ce cas il peut être prévu d'introduire, dans chaque dit paquet de données à synchroniser, une information d'identification permettant d'associer ledit paquet au signal de synchronisation correspondant.
- En variante, ledit ou chaque terminal d'émission peut émettre une pluralité de paquets de données à synchroniser, ainsi qu'un signal de synchronisation tous les N dits paquets de données avec N>1, lesdits paquets de données à synchroniser étant alors émis à une cadence qui est sensiblement constante au moins sur une période comprise entre l'émission de deux signaux de synchronisation consécutifs. Dans ce cas il peut être prévu d'introduire, dans chaque paquet de données en correspondance duquel un signal de synchronisation est émis, une information d'identification permettant d'associer ledit paquet au signal de synchronisation correspondant. Il peut aussi être prévu d'introduire, dans chaque dit paquet de données à synchroniser, une information permettant de déterminer l'ordre dans lequel lesdits paquets ont été émis.
- En variante, ledit ou chaque terminal d'émission peut émettre un seul signal de synchronisation au cours d'une session de transmission de données. Ce mode de réalisation suppose que les fréquences d'horloge du ou des terminaux d'émission et du terminal de réception soient identiques, ou en tout cas connues et présentant des dérives négligeables.
- Ledit ou chaque paquet de données à synchroniser peut notamment représenter un signal acquis par un capteur ou ensemble de capteurs associé audit ou à un dit terminal d'émission. Plus particulièrement, ledit ou chaque paquet de données à synchroniser peut représenter un signal neuronal généré par un patient en réponse à un stimulus sensoriel, ladite étape d) de synchronisation comportant la localisation dudit ou de chaque dit paquet de données et dudit stimulus sur une base de temps commune.

Un autre objet de l'invention est un système d'acquisition de données défini selon la revendication 11.

Selon différents modes de réalisation du système de l'invention :
- Tant ledit canal de transmission que ledit canal de transmission auxiliaire peuvent être des canaux de transmission sans fil.
- Ledit ou chaque terminal d'émission peut être adapté pour émettre un dit signal de synchronisation en correspondance de chaque dit paquet de données.
- En variante, ledit ou chaque terminal d'émission peut être adapté pour émettre une pluralité de paquets de données à synchroniser ainsi qu'un signal de synchronisation tous les N dits paquets de données, avec N>1, lesdits paquets de données à synchroniser étant émis à une cadence qui est sensiblement constante au moins sur une période comprise entre l'émission de deux signaux de synchronisation consécutifs.
- Le système d'acquisition peut comporter également un capteur ou ensemble de capteurs associé audit ou à chaque dit termina! d'émission, ledit ou chaque paquet de données émis par ledit ou chaque terminal d'émission représentant un signal acquis par le capteur ou ensemble de capteurs correspondant.
- Le système d'acquisition peut comporter également un dispositif de stimulation sensorielle d'un patient, et en outre :
- ledit ou chaque capteur ou ensemble de capteurs peut être adapté pour acquérir des signaux neuronaux représentatif de la réponse d'un patient à un stimulus sensoriel engendré par ledit dispositif de stimulation ; et
- ledit terminal de réception peut être adapté pour localiser ledit ou chaque dit paquet de données et ledit stimulus sur une base de temps commune.

D'autres caractéristiques, détails et avantages de l'invention ressortiront à la lecture de la description faite en référence aux dessins annexés donnés à titre d'exemple, dans lesquels :
- La figure 3 illustre de manière générale le principe à la base du procédé de synchronisation de données objet de l'invention ;
- Les figures 4 à 6 sont des schémas fonctionnels de systèmes d'acquisition de données selon différents modes de réalisation de l'invention ; et
- Les figures 7 et 8 illustrent la validation expérimentale d'un procédé de synchronisation des données selon l'invention.

Comme expliqué plus haut, un canal de transmission sans fil robuste présente une latence variable en raison de l'utilisation d'un protocole de communication destiné à assurer l'intégrité des données transmises (détection et correction d'erreurs, envoi d'accusés de réception...) et des délais internes à l'unité de traitement réalisant l'acquisition desdites données. Afin de procéder à la synchronisation des signaux transmis, et donc de les situer sur une base des temps commune, la présente invention prévoit l'utilisation d'un canal de transmission auxiliaire, également sans fil mais présentant une latence maîtrisée. Ce canal auxiliaire ne doit pas nécessairement présenter une robustesse et/ou une capacité élevées car il n'est utilisé que pour transmettre un signal de synchronisation - le plus souvent un simple code - en même temps qu'un paquet de données à synchroniser (ou, plus généralement, avec une relation temporelle connue avec l'instant d'émission de ce paquet). Comme la latence du canal auxiliaire est connue, la connaissance de l'instant de réception du signal de synchronisation permet de déterminer son instant d'émission, et donc celui du paquet de données.

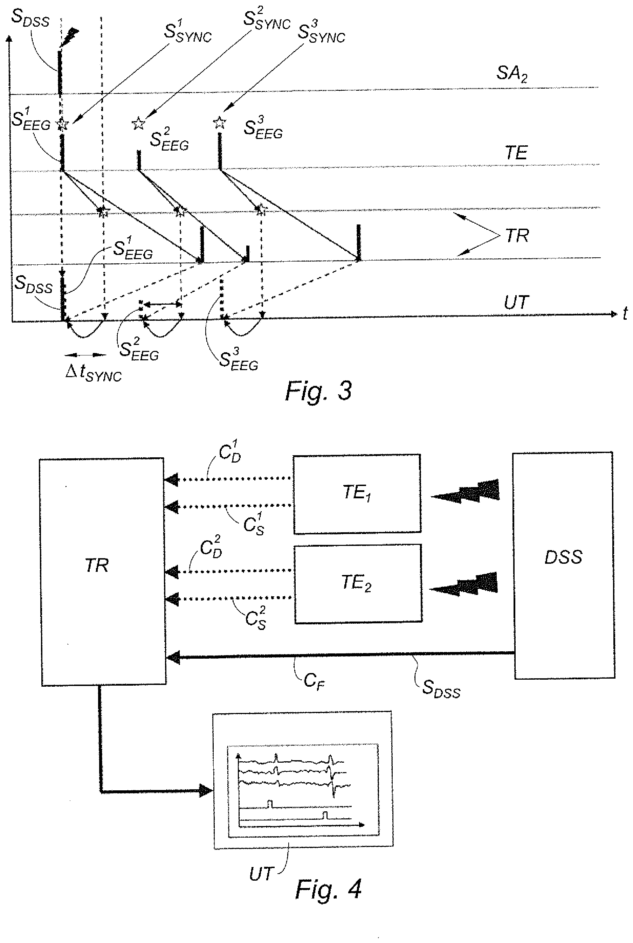

Ce principe de fonctionnement est illustré sur la figure 3. Cette figure fait référence à un système semblable à celui représenté sur la figure 2, complété cependant par l'adjonction du canal auxiliaire entre le terminal d'émission TE et le terminal de réception TR du système d'acquisition SA₁. On remarquera que le deuxième système d'acquisition SA₂ est relié à l'unité de traitement UT par une connexion filaire, et présente donc une base de temps commune avec elle (temps « t »).

La génération d'un stimulus sensoriel s'accompagne par la réception, par le système d'acquisition SA₂, d'un signal S_{DSS}. Ce stimulus déclenche chez le patient P une réponse, qui est échantillonnée et numérisée. Cette réponse correspond par exemple à un signal mesuré par une ou plusieurs électrodes. L'échantillonnage temporel de cette réponse génère trois paquets de données : S_{EEG}¹, S_{EEG}² et S_{EEG}³. Par paquet de données, on entend l'information résultant de l'échantillonnage des signaux produits par les électrodes en un instant donné. D'une façon générale, un paquet de données englobe des signaux transmis simultanément par le système d'acquisition.

Le système d'acquisition SA₁ génère ces trois paquets de données et les retransmets sur le canal de transmission à latence non maîtrisée ; en même temps, il génère trois signaux de synchronisation S_{SYNC}¹, S_{SYNC}² et S_{SYNC}³ et les transmets sur le canal auxiliaire à latence maîtrisée. En général, le canal auxiliaire n'aura ni une capacité ni une robustesse suffisante pour assurer la transmission des paquets de données, et cela justement afin de pouvoir présenter une latence aussi constante que possible. Le terminal de réception TR reçoit les signaux de synchronisation avec un délai connu, Δt, et les paquets de données avec des délais inconnus et différents entre eux ; ces signaux sont ensuite transmis - avec une information indicative de leur instant de réception - à l'unité de traitement UT. Cette dernière dispose ainsi de tous les éléments lui permettant d'effectuer la synchronisation ; la latence Δt du canal auxiliaire étant connue, l'instant d'émission des signaux synchronisation S_{SYNC}¹, S_{SYNC}² et S_{SYNC}³ peut être calculée ; on sait en outre que les paquets de données S_{EEG}¹, S_{EEG}² et S_{EEG}³ ont été émis en même temps que lesdits signaux de synchronisation. Ces trois paquets de données peuvent ainsi être placés sur une base de temps commune, alors même que la transmission sur le canal de communication sans fil avait altéré la relation temporelle existante entre eux. Le signal S_{DSS} peut être placé sur la même base de temps sans difficulté particulière, car il a été acquis par l'intermédiaire d'une connexion filaire. De cette façon, on peut déterminer l'instant d'émission de chaque paquet de données S_{EEG}¹, S_{EEG}² et S_{EEG}³ par rapport au signal de stimulus S_{DSS}

On remarquera que la mise en oeuvre du procédé de l'invention ne nécessite pas de modification profonde du système d'acquisition de la figure 2, mais seulement l'ajout d'un canal de communication sans fil auxiliaire à latence maîtrisée.

Dans le cas de la figure 3, un signal de synchronisation est émis simultanément à chaque paquet de données S_{EEG}. Comme la perte d'un signal de synchronisation ne peut pas être exclue (le canal auxiliaire présente une robustesse relativement faible), il est opportun que chaque paquet de données contienne une information d'identification, ou horodatage, permettant de l'associer au signal de synchronisation correspondant. Ainsi, la perte d'un signal de synchronisation ne fait qu'introduire une incertitude sur l'instant d'émission d'un seul paquet de données, mais ne perturbe pas fondamentalement le processus de synchronisation. Ce mode de réalisation convient aux cas où les paquets de données sont transmis à une cadence irrégulière, en correspondance d'événements non prévisibles.

Si les paquets de données sont émis à une cadence régulière - par exemple s'ils sont obtenus par échantillonnage à une fréquence fₛ constante et numérisation d'un signal analogique - il sera généralement préférable d'émettre un signal de synchronisation tous les N paquets avec N>1. Par exemple, dans le cas d'une fréquence d'échantillonnage fₛ=1 kHz, on pourra prendre N=60.000, ce qui correspond à l'émission d'un signal de synchronisation par minute. En fait, la valeur maximale admissible de N dépend essentiellement de la stabilité de la fréquence d'échantillonnage fₛ, qui est supposée constante entre deux signaux de synchronisation.

Dans ce deuxième mode de réalisation, il est opportun que les paquets de données contiennent :
- une information permettant d'identifier ceux, parmi lesdits paquets, qui ont été transmis en même temps qu'un signal de synchronisation ; et/ou
- l'ordre d'émission desdits paquets.

Par exemple, chaque paquet peut contenir un numéro de comptage qui est incrémenté à chaque émission jusqu'à une valeur maximale de N-1 avant d'être réinitialisé à zéro, les paquets portant le numéro zéro correspondant à l'émission d'un signal de synchronisation.

Ainsi, la perte d'un signal de synchronisation est détectée aisément, connaissant la fréquence à laquelle le signal de synchronisation doit être reçu. En outre, la synchronisation des paquets de données peut s'effectuer malgré cette perte, en supposant que la fréquence fₛ soit restée sensiblement constante.

En fait, si la fréquence d'échantillonnage fₛ et les fréquences d'horloge du système d'acquisition SA₂ et du terminal de réception TR sont connues et stables, il suffit de synchroniser les origines des bases de temps par l'envoi d'un seul signal de synchronisation au cours de chaque session d'acquisition de données. Dans la plupart des cas, cependant, on ne pourra pas ignorer les dérives des fréquences d'horloge, qui dans un système d'acquisition de signaux médicaux sont typiquement de l'ordre de 20 ppm (parties par million) = 72 ms/heure, et il faudra donc avoir recours à une émission périodique de signaux de synchronisation afin de corriger ces dérives.

Le procédé de synchronisation ainsi décrit peut être utilisé pour synchroniser les données provenant d'une pluralité de terminaux d'émission sans connexion directe entre eux, par exemple associés à une pluralité de casques d'EEG sans fil portés par des patients respectifs. De même, le dispositif de stimulation sensorielle DSS, lorsqu'il est présent, peut être connecté à l'unité de traitement par un canal sans fil.

Grâce à la synchronisation, il n'est pas nécessaire d'assurer un démarrage simultané des différents systèmes d'acquisition et de l'éventuel dispositif de stimulation sensorielle.

Un autre avantage du procédé est que la qualité du lien de communication sans fil n'influence pas la synchronisation des données.

La figure 4 illustre un système d'acquisition selon un mode de réalisation de l'invention, comportant deux terminaux d'acquisition TE₁ et TE₂ ; connectés à des casques de EEG (non représentés) et reliés à un terminal de réception unique TR par des connexions sans fil. Plus précisément, chaque terminal d'émission est relié au terminal de réception par un canal de transmission des données C_{D}¹, C_{D}², à latence non maîtrisée, et par un canal auxiliaire, ou de synchronisation, C_{S}¹, C_{S}², à latence maîtrisée. Un dispositif de stimulation sensorielle DSS est relié au terminal de réception TR par un canal de transmission filaire C_{F} ; le terminal de réception TR est relié par un autre canal de transmission filaire à l'unité de traitement des données UT.

Les caractéristiques principales du système sont les suivantes :
- fréquence d'échantillonnage des signaux d'EEG : 1 kHz ;
- taille de chaque échantillon: 384 bits (32 voies, 12 bits/voie) ;
- Canaux de communication de données en bande MICS (402-405 MHz), protocole de communication propriétaire Zarlink, débit 400 kbit/s ;
- Canaux de synchronisation: lien infrarouge, protocole IrDA ;
- Emission d'un signal de synchronisation tous les N=2¹⁶ échantillons, donc environ toutes les minutes.

Chaque échantillon envoyé sur le canal de communication de données est identifié avec un numéro incrémenté à chaque échantillon, qui est réinitialisé à zéro quand il atteint la valeur 2¹⁶-1.

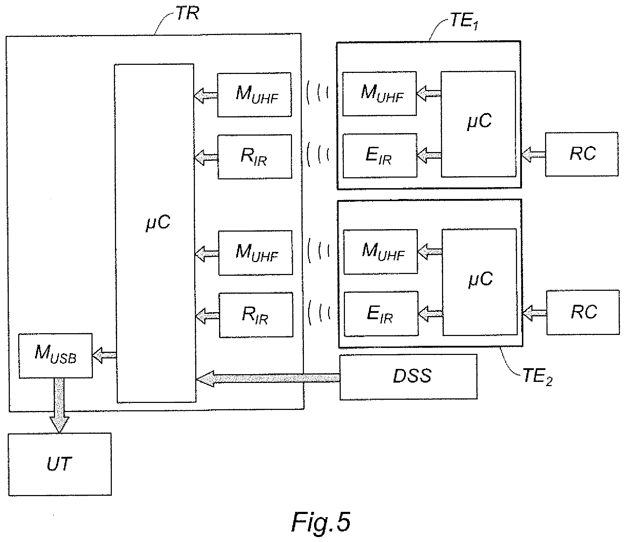

La figure 5 illustre plus en détail l'architecture du système de la figure 4. On peut voir sur cette figure que :
- Chaque terminal d'émission comprend un microcontrôleur µC qui reçoit des signaux d'un ensemble de capteurs RC (par exemple, des capteurs d'EEG), les échantillonne et les convertit au format numérique sous la forme de paquets de données. Le microcontrôleur génère également les signaux de synchronisation.
- Chaque canal de transmission de données est réalisé par l'intermédiaire de transpondeurs UHF du commerce M_{UHF}, intégrant un contrôle de flux automatique, la correction des erreurs et la gestion des retransmissions. Ces transpondeurs, présents du côté émission et du côté réception, permettent de garantir l'intégrité des données et un débit élevé, au prix d'une latence variable.
- Les canaux auxiliaires de synchronisation, unidirectionnels, sont réalisés au moyen d'émetteurs (E_{IR}) et récepteurs (R_{IR}) infrarouges.
- Le terminal de réception comprend à son tour un microcontrôleur µC, et communique avec une unité de traitement des données (UT) au moyen d'un module USB, M_{USB}. Ce terminal comprend également une unité qui communique avec le système de génération de stimuli DSS.

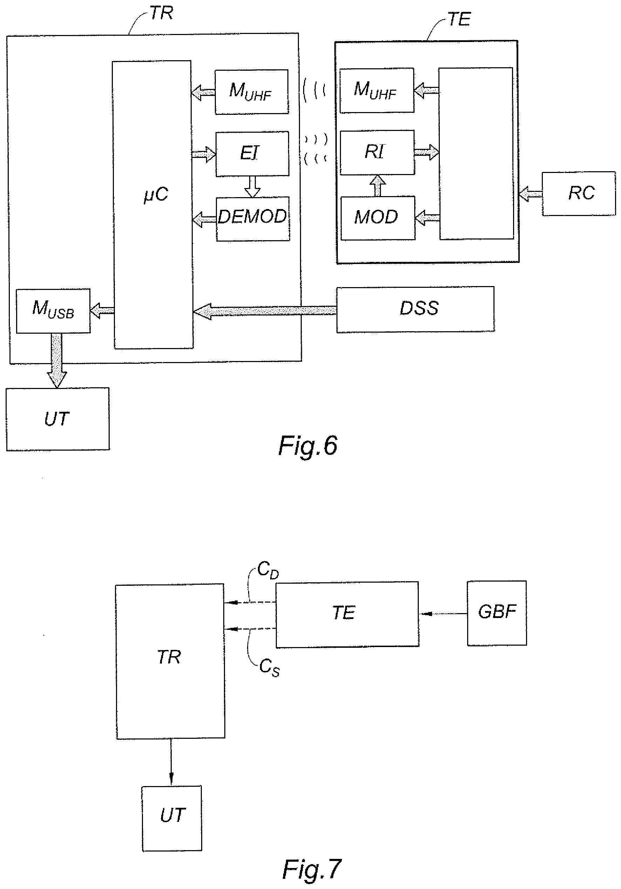

La figure 6 illustre l'architecture d'un système d'acquisition des données utilisant un terminal d'émission implanté dans le corps (par exemple dans le crâne) d'un patient.

Le canal de transmission des données C_{D} est réalisé par l'intermédiaire de transpondeurs UHF, comme dans le mode de réalisation de la figure 5. Pour le canal de synchronisation C_{S}, en revanche, on exploite un couplage inductif assurant la téléalimentation de l'implant, selon le principe des étiquettes d'identification par radiofréquence (RFID). Ce couplage est réalisé au moyen d'un émetteur inductif (antenne) EI situé du côté du terminal de réception TR et un récepteur inductif RI (boucle) situé du côté du terminal d'émission. Un modulateur de charge MOD module la charge vue par l'émetteur inductif selon une fréquence porteuse et un encodage Manchester, réalisant ainsi une modulation d'amplitude sur le lien inductif. L'antenne émettrice EI est connectée à un module de démodulation DEMOD situé sur le terminal de réception qui reconstitue le message d'origine. La transmission de ce message s'effectuant selon une séquence prédéfinie et à latence constante, il est possible de réaliser la synchronisation des acquisitions provenant du système implanté avec d'autres systèmes d'acquisition connectés au même terminal de réception et/ou avec le dispositif de stimulation.

Le procédé de l'invention a été testé au moyen du montage expérimental de la figure 7. Ce montage comprend un générateur basse fréquence GBF utilisé pour générer des impulsions à une fréquence de répétition f_{R}=1 Hz. Ces impulsions ont été échantillonnées à une fréquence d'échantillonnage fₛ dont la valeur nominale fₛ⁰ était de 600 Hz et la valeur réelle fₛ était réglée à 1 kHz afin de simuler l'effet d'une dérive de fréquence. Les signaux échantillonnés ont été transmis d'un terminal d'émission TE vers un terminal d'acquisition TR, relié à un ordinateur UT, au moyen d'une liaison sans fil hertzienne en bande MICS (402-405 MHz) utilisant le protocole propriétaire Zarlink, à un débit 400 kbit/s (canal de données C_{D}, présentant une latence non maîtrisée). Un signal de synchronisation S_{SYNC} a été généré tous les 2048 échantillons, et transmis du terminal d'émission vers par le terminal de réception via une liaison unidirectionnelle infrarouge (protocole IrDA) à latence maîtrisée, C_{S}.

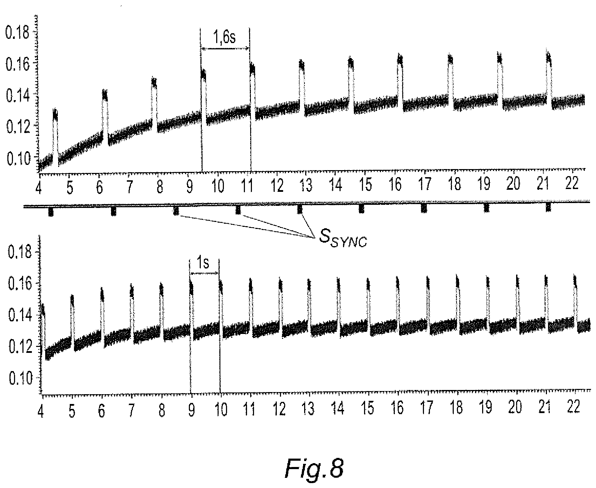

L'unité de traitement des données est programmée pour acquérir les données transmises sur le canal C_{D} et reconstituer le signal impulsionnel en partant de l'hypothèse qu'il ait été échantillonné à la fréquence nominale fₛ⁰=600 Hz, et pas à la fréquence réelle fₛ=1 kHz. En l'absence de synchronisation, cela conduit à un signal reconstitué présentant une fréquence de répétition des impulsions f'_{R}=0,6 Hz (au lieu de f_{R}=1 Hz), deux fronts montants desdites impulsions étant séparés de 1000/600≈1,6 seconde. Le panneau supérieur de la figure 8 montre la forme d'onde du signal impulsionnel reconstitué par l'unité de traitement des donnés dans ces conditions. Le panneau inférieur de cette même figure montre que l'application d'un procédé de synchronisation selon l'invention permet une reconstitution correcte du signal impulsionnel (intervalle de 1 s entre deux fronts montants consécutifs), malgré le fait que la fréquence d'échantillonnage réelle soit très différente de la fréquence d'échantillonnage nominale. Le résultat obtenu est d'autant plus significatif que l'erreur de fréquence d'échantillonnage a volontairement été choisi beaucoup plus important de ce qu'on peut raisonnablement s'attendre dans la plupart des applications réelles (660.000 parties par million - ppm).

La synchronisation permet donc :
- un recalage temporel du signal numérique transmis, qui est « aligné » avec le signal de synchronisation ;
- un calcul de la fréquence réelle d'échantillonnage (l'unité de traitement connait le nombre N d'échantillons compris entre deux signaux de synchronisation - ici, 2048 ; la fréquence fₛ est donc obtenue en divisant par N l'intervalle temporel entre deux signaux de synchronisation) ; et
- le cas échéant, un rééchantillonnage du signal reçu à la fréquence nominale fₛ.

L'invention a été décrite en référence à une application particulière, l'acquisition de signaux d'électroencéphalographie (EEG) en réponse à un stimulus sensoriel. Elle n'est cependant pas limitée à cette seule application, mais concerne tous les cas où il est nécessaire de synchroniser des données transmises via des canaux (non nécessairement sans fil) à latence non maîtrisée.

## Revendications

1. Procédé de synchronisation de données transmises entre au moins un terminal d'émission (TE) et un terminal de réception (TR) via un canal de transmission (C_{D}) à latence non maîtrisée et variable, comportant les étapes consistant à :
a) au niveau dudit ou de chaque terminal d'émission, générer au moins un signal de synchronisation (S_{SYNC}) ayant une relation temporelle connue avec l'instant d'émission d'au moins un paquet (S_{EEG}) de données à synchroniser ; Le procédé **caractérisé par** les étapes suivantes:
b) émettre ledit ou chaque paquet de données à synchroniser sur ledit canal de transmission à latence non maîtrisée et variable, et en même temps ledit ou chaque signal de synchronisation sur un canal de transmission auxiliaire (C_{S}) à latence maîtrisé entre le terminal d'émission (TE) et le terminal de réception (TR) ;
c) au niveau dudit terminal de réception, recevoir ledit ou chaque paquet de données à synchroniser et ledit ou chaque signal de synchronisation ; et
d) synchroniser sur une base de temps commune ledit ou chaque paquet de données reçu par ledit terminal de réception au moyen dudit signal de synchronisation et de ladite latence du canal de transmission auxiliaire.

2. Procédé de synchronisation de données selon la revendication 1, dans lequel tant ledit canal de transmission que ledit canal de transmission auxiliaire sont des canaux de transmission sans fil.

3. Procédé de synchronisation de données selon l'une des revendications précédentes, dans lequel ledit ou chaque terminai d'émission émet une pluralité de paquets de données à synchroniser, ainsi qu'un dit signal de synchronisation en correspondance de chaque dit paquet de données à synchroniser.

4. Procédé de synchronisation de données selon la revendication 3, dans lequel on introduit dans chaque dit paquet de données à synchroniser une information d'identification permettant d'associer ledit paquet au signal de synchronisation correspondant.

5. Procédé de synchronisation de données selon l'une des revendications 1 ou 2, dans lequel ledit ou chaque terminal d'émission émet une pluralité de paquets de données à synchroniser, ainsi qu'un signal de synchronisation tous les N dits paquets de données avec N>1, lesdits paquets de données à synchroniser étant émis à une cadence qui est sensiblement constante au moins sur une période comprise entre l'émission de deux signaux de synchronisation consécutifs.

6. Procédé de synchronisation de données selon la revendication 5, dans lequel on introduit, dans chaque paquet de données en correspondance duquel un signal de synchronisation est émis, une information d'identification permettant d'associer ledit paquet au signal de synchronisation correspondant.

7. Procédé de synchronisation de données selon l'une des revendications 5 ou 6 dans lequel on introduit, dans chaque dit paquet de données à synchroniser, une information permettant de déterminer l'ordre dans lequel lesdits paquets ont été émis.

8. Procédé de synchronisation de données selon l'une des revendications 1 ou 2, dans lequel ledit ou chaque terminal d'émission émet un seul signal de synchronisation au cours d'une session de transmission de données.

9. Procédé de synchronisation de données selon l'une des revendications précédentes, dans lequel ledit ou chaque paquet de données à synchroniser représente un signal acquis par un capteur ou ensemble de capteurs associé audit ou à un dit terminal d'émission.

10. Procédé de synchronisation de données selon la revendication 9, dans lequel ledit ou chaque paquet de données à synchroniser représente un signal neuronal généré par un patient en réponse à un stimulus sensoriel, ladite étape d) de synchronisation comportant la localisation dudit ou de chaque dit paquet de données et dudit stimulus sur une base de temps commune.

11. Système d'acquisition de données comportant :
- au moins un terminal d'émission (TE), adapté pour transmettre au moins un paquet de données (S_{EEG}) sur un canal de transmission (C_{D}) ; et
- un terminal de réception (TR), adapté pour recevoir ledit ou chaque paquet de données via ledit canal de transmission ;
ledit canal de transmission étant à latence non maîtrisée et variable ; **caractérisé en ce que** :
- ledit ou chaque terminal d'émission est adapté pour émettre, en même temps que ledit ou chaque paquet de données, sur un canal de transmission auxiliaire (C_{S}) à latence maîtrisée, au moins un signal de synchronisation (S_{SYNC}) ayant une relation temporelle connue avec l'instant d'émission dudit ou d'au moins un dit paquet de données ; et **en ce que**
- ledit terminal de réception est adapté pour recevoir ledit ou chaque paquet de données à synchroniser et ledit ou chaque signal de synchronisation, et pour synchroniser sur une base de temps commune ledit ou chaque paquet de données reçu au moyen dudit signal de synchronisation et de ladite latence du canal de transmission auxiliaire.

12. Système d'acquisition de données selon la revendication 11, dans lequel tant ledit canal de transmission que ledit canal de transmission auxiliaire sont des canaux de transmission sans fil.

13. Système d'acquisition de données selon l'une des revendications 11 ou 12 dans lequel ledit ou chaque terminal d'émission est adapté pour émettre un dit signal de synchronisation en correspondance de chaque dit paquet de données.

14. Système d'acquisition de données selon l'une des revendications 11 ou 12 dans lequel ledit ou chaque terminal d'émission est adapté pour émettre une pluralité de paquets de données à synchroniser ainsi qu'un signal de synchronisation tous les N dits paquets de données, avec N>1, lesdits paquets de données à synchroniser étant émis à une cadence qui est sensiblement constante au moins sur une période comprise entre l'émission de deux signaux de synchronisation consécutifs.

15. Système d'acquisition de données selon l'une des revendications 11 à 14, comportant également un capteur ou ensemble de capteurs (RC) associé audit ou à chaque dit terminal d'émission, ledit ou chaque paquet de données émis par ledit ou chaque terminal d'émission représentant un signal acquis par le capteur ou ensemble de capteurs correspondant.

16. Système d'acquisition de données selon la revendication 15 comportant également un dispositif de stimulation sensorielle (DSS) d'un patient (P), dans lequel :
- ledit ou chaque capteur ou ensemble de capteurs est adapté pour acquérir des signaux neuronal représentatif de la réponse d'un patient à un stimulus sensoriel engendré par ledit dispositif de stimulation ; et
- ledit terminal de réception est adapté pour localiser ledit ou chaque dit paquet de données et ledit stimulus sur une base de temps commune.

## Patentansprüche

1. Synchronisationsverfahren für Daten, die zwischen mindestens einem Sendeendgerät (TE) und einem Empfangsendgerät (TR) über einen Übertragungskanal (C_{D}) mit unkontrollierter und variabler Verzögerung übertragen werden, einschließend die Schritte bestehend aus:
a) hinsichtlich des oder jedes Sendeendgeräts, Erzeugen mindestens eines Synchronisationssignals (S_{SYNC}), das eine bekannte zeitliche Beziehung zum Sendezeitpunkt mindestens eines zu synchronisierenden Datenpakets (S_{EEG}) aufweist; wobei das Verfahren durch die folgenden Schritte gekennzeichnet ist:
b) Senden des oder jedes zu synchronisierenden Datenpakets auf dem Übertragungskanal mit unkontrollierter und variabler Verzögerung und gleichzeitig
des oder jedes Synchronisationssignals auf einem Hilfsübertragungskanal (C_{S}) mit kontrollierter Verzögerung zwischen dem Sendeendgerät (TE) und dem Empfangsendgerät (TR);
c) hinsichtlich des Empfangsendgeräts, Empfangen des oder jedes zu synchronisierenden Datenpakets und des oder jedes Synchronisationssignals; und
d) Synchronisieren auf einer Basis gemeinsamer Zeit des oder jedes vom Empfangsendgerät empfangenen Datenpakets mithilfe des Synchronisationssignals und der Verzögerung des Hilfsübertragungskanals.

2. Synchronisationsverfahren für Daten nach Anspruch 1, wobei sowohl der Übertragungskanal als auch der Hilfsübertragungskanal drahtlose Übertragungskanäle sind.

3. Synchronisationsverfahren für Daten nach einem der vorstehenden Ansprüche, wobei das oder jedes Sendeendgerät eine Vielzahl von zu synchronisierenden Datenpaketen sowie ein sogenanntes Synchronisationssignal in Entsprechung mit jedem sogenannten zu synchronisierenden Datenpaket sendet.

4. Synchronisationsverfahren für Daten nach Anspruch 3, wobei in jedes zu synchronisierende Datenpaket eine Identifikationsinformation eingegeben wird, die es erlaubt, das Paket mit dem entsprechenden Synchronisationssignal zu verknüpfen.

5. Synchronisationsverfahren für Daten nach einem der Ansprüche 1 oder 2, wobei das oder jedes Sendeendgerät eine Vielzahl von zu synchronisierenden Datenpaketen sowie alle N sogenannten Datenpakete, mit N > 1, ein Synchronisationssignal sendet, wobei die zu synchronisierenden Datenpakete in einem Takt gesendet werden, der im Wesentlichen über mindestens eine Periode zwischen der Sendung zweier aufeinanderfolgender Synchronisationssignale konstant ist.

6. Synchronisationsverfahren für Daten nach Anspruch 5, wobei in jedes Datenpaket, in Entsprechung dessen ein Synchronisationssignal gesendet wird, eine Identifikationsinformation eingegeben wird, die es erlaubt, das Paket mit dem entsprechenden Synchronisationssignal zu verknüpfen.

7. Synchronisationsverfahren für Daten nach einem der Ansprüche 5 oder 6, wobei in jedes sogenannte zu synchronisierende Datenpaket eine Information eingegeben wird, die es erlaubt, die Reihenfolge, in der die Pakete gesendet wurden, zu bestimmen.

8. Synchronisationsverfahren für Daten nach einem der Ansprüche 1 oder 2, wobei das oder jedes Sendeendgerät im Laufe einer Datenübertragungssitzung ein einziges Synchronisationssignal sendet.

9. Synchronisationsverfahren für Daten nach einem der vorstehenden Ansprüche, wobei das oder jedes zu synchronisierende Datenpaket ein Signal darstellt, das von einem Sensor oder einer Sensoranordnung erfasst wurde, der/die mit dem oder mit einem sogenannten Sendeendgerät verknüpft ist.

10. Synchronisationsverfahren für Daten nach Anspruch 9, wobei das oder jedes zu synchronisierende Datenpaket ein neuronales Signal darstellt, das von einem Patienten als Reaktion auf einen sensoriellen Stimulus erzeugt wird, wobei der Schritt d) der Synchronisierung die Lokalisierung des oder jedes sogenannten Datenpakets und des Stimulus auf einer Basis gemeinsamer Zeit einschließt.

11. Erfassungssystem für Daten, einschließend:
- mindestens ein Sendeendgerät (TE), das angepasst ist, um mindestens ein Datenpaket (S_{EEG}) auf einem Übertragungskanal (C_{D}) zu übertragen; und
- ein Empfangsendgerät (TR), das angepasst ist, um das oder jedes Datenpaket über den Übertragungskanal zu empfangen;
wobei der Übertragungskanal mit einer unkontrollierten und variablen Verzögerung ist; **dadurch gekennzeichnet, dass**:
- das oder jedes Sendeendgerät angepasst ist, um gleichzeitig mit dem oder jedem Datenpaket auf einem Hilfsübertragungskanal (C_{S}) mit kontrollierter Verzögerung mindestens ein Synchronisationssignal (S_{SYNC}) zu senden, das eine bekannte zeitliche Beziehung zum Sendezeitpunkt des oder mindestens eines sogenannten Datenpakets aufweist; und dadurch, dass
- das Empfangsendgerät angepasst ist, um das oder jedes zu synchronisierende Datenpaket und das oder jedes Synchronisationssignal zu empfangen, und um auf einer Basis gemeinsamer Zeit das oder jedes empfangene Datenpaket mithilfe des Synchronisationssignals und der Verzögerung des Hilfsübertragungskanals zu synchronisieren.

12. Erfassungssystem für Daten nach Anspruch 11, wobei sowohl der Übertragungskanal als auch der Hilfsübertragungskanal drahtlose Übertragungskanäle sind.

13. Erfassungssystem für Daten nach einem der Ansprüche 11 oder 12, wobei das oder jedes Sendeendgerät angepasst ist, um ein sogenanntes Synchronisationssignal in Entsprechung mit jedem sogenannten Datenpaket zu senden.

14. Erfassungssystem für Daten nach einem der Ansprüche 11 oder 12, wobei das oder jedes Sendeendgerät angepasst ist, um eine Vielzahl von zu synchronisierenden Datenpaketen sowie alle N sogenannten Datenpakete, mit N > 1, ein Synchronisationssignal zu senden, wobei die zu synchronisierenden Datenpakete in einem Takt gesendet werden, der im Wesentlichen über mindestens eine Periode zwischen der Sendung zweier aufeinanderfolgender Synchronisationssignale konstant ist.

15. Erfassungssystem für Daten nach einem der Ansprüche 11 bis 14, das ebenfalls einen Sensor oder eine Sensoranordnung (RC) einschließt, der/die mit dem oder mit jedem sogenannten Sendeendgerät verknüpft ist, wobei das oder jedes Datenpaket, das durch das oder jedes Sendeendgerät gesendet wird, ein Signal darstellt, das durch den entsprechenden Sensor oder die entsprechende Sensoranordnung erfasst wurde.

16. Erfassungssystem für Daten nach Anspruch 15, das ebenfalls eine Vorrichtung zur sensoriellen Stimulation (DSS) eines Patienten (P) einschließt, wobei:
- der/die oder jeder/jede Sensor oder Sensoranordnung angepasst ist, um neuronale Signale zu erfassen, die repräsentativ sind für die Reaktion eines Patienten auf einen sensoriellen Stimulus, der durch die Vorrichtung zur Stimulation hervorgerufen wird; und
- das Empfangsendgerät angepasst ist, um das oder jedes sogenannte Datenpaket und den Stimulus auf einer Basis gemeinsamer Zeit zu lokalisieren.

## Claims

1. Method for synchronising data transmitted between at least one transmitting terminal (TE) and one receiving terminal (TR) through a communication channel (C_{D}) with uncontrolled and variable latency, comprising the following steps:
a) at the level of said or of each transmitting terminal, generating at least one synchronisation signal (S_{SYNC}) having a known temporal relation to the moment of emission of at least one packet (S_{EEG}) of data to be synchronised; the method being **characterised by** the following steps:
b) emitting said or each packet of data to be synchronised on said communication channel with uncontrolled and variable latency, and simultaneously, said or each synchronisation signal on an auxiliary communication channel (C_{S}) with controlled latency between the transmitting terminal (TE) and the receiving terminal (TR);
c) at the level of said receiving terminal, receiving said or each packet of data to be synchronised and said or each synchronisation signal; and
d) synchronising on a shared time basis said or each data packet received by said receiving terminal by means of said synchronisation signal and of said latency of the auxiliary communication channel.

2. Method for synchronising data according to claim 1, wherein both said communication channel and said auxiliary communication channel are wireless communication channels.

3. Method for synchronising data according to one of the previous claims, wherein said or each transmitting terminal emits a plurality of packets of data to be synchronised, as well as one said synchronisation signal corresponding to each said packet of data to be synchronised.

4. Method for synchronising data according to claim 3, wherein identification information is introduced in each packet of data to be synchronised enabling associating said packet with the corresponding synchronisation signal.

5. Method for synchronising data according to one of the claims 1 or 2, wherein said or each transmitting terminal emits a plurality of packets of data to be synchronised, as well as a synchronisation signal every N said packets of data, with N>1, said packets of data to be synchronised being emitted at a frequency that is substantially constant at least in a period covering the emission of two consecutive synchronisation signals.

6. Method for synchronising data according to claim 5, wherein identification information is introduced, in each packet of data, for which a corresponding synchronisation signal is emitted, enabling associating said packet with the corresponding synchronisation signal.

7. Method for synchronising data according to one of the claims 5 or 6, wherein information is introduced, in each said packet of data to be synchronised, enabling determining the order in which said packets were emitted.

8. Method for synchronising data according to one of the claims 1 or 2, wherein said or each transmitting terminal emits a single synchronisation signal during a data transmission session.

9. Method for synchronising data according to one of the preceding claims, wherein said or each packet of data to be synchronised represents a signal acquired by a sensor or by a plurality of sensors associated with said or with one said transmitting terminal.

10. Method for synchronising data according to claim 9, wherein said or each packet of data to be synchronised represents a neuronal signal generated by a patient in response to a sensory stimulus, said synchronisation step d) comprising localising said or each packet of data and said stimulus on a shared time basis.

11. Data acquisition system comprising:
- at least one transmitting terminal (TE) configured to transmit at least one data packet (S_{EEG}) on a communication channel (C_{D}); and
- a receiving terminal (TR) configured to receive said or each data packet through said communication channel;
said channel having uncontrolled and variable latency; **characterised in that**:
- said or each communication channel is configured to emit, at the same time as said or each data packet, on an auxiliary communication channel (C_{S}) with controlled latency, at least one synchronisation signal (S_{SYNC}) having a known temporal relation to the moment of emission of said or at least one said data packet; and **in that**
- said receiving terminal is configured to receive said or each packet of data to be synchronised and said or each synchronisation signal, and to synchronise on a shared time basis said or each received data packet by means of said synchronisation signal and said latency of the auxiliary communication channel.

12. Data acquisition system according to claim 11, wherein both said communication channel and said auxiliary communication channel are wireless communication channels.

13. Data acquisition system according to one of the claims 11 or 12, wherein said or each transmitting terminal is configured to emit a said synchronisation signal corresponding to each said data packet.

14. Data acquisition system according to one of the claims 11 or 12, wherein said or each transmitting terminal is configured to emit a plurality of packets of data to be synchronised as well as a synchronisation signal every N said data packets, with N>1, said packets of data to be synchronised being emitted at a frequency that is substantially constant at least on a period covering the emission of two consecutive synchronisation signals.

15. Data acquisition system according to one of the claims 11 to 14, also comprising a sensor or a plurality of sensors (RC) associated with said or each said transmitting terminal, said or each data packet emitted by said or each transmission terminal representing a signal acquired by the corresponding sensor or plurality of sensors.

16. Data acquisition system according to claim 15, also comprising a sensory stimulation device (DSS) of a patient (P), wherein:
- said or each sensor or plurality of sensors is configured to acquire neuronal signals representative of the response of a patient to a sensory stimulus generated by said stimulation device; and
- said receiving terminal is adapted to localise said or each data packet and said stimulus on a shared time basis.
